# FASCICULE DE BREVET EUROPEEN

(11) **EP 3 270 868 B1**
(45) Date de publication et mention de la délivrance du brevet: **09.01.2019**
(21) Numéro de dépôt: 15714412.2
(22) Date de dépôt: 19.03.2015
(51) Int. Cl.: A61J 3/00, B05B 13/02, B01J 2/00, A23G 3/20, A23G 3/26, A61K 9/28

(54) **DISPOSITIF D'ENROBAGE DE COMPRIMÉS PHARMACEUTIQUES**
VORRICHTUNG ZUM BESCHICHTEN VON PHARMAZEUTISCHE PILLEN
DEVICE FOR COATING PHARMACEUTICAL TABLETS

(43) Date de publication de la demande: 24.01.2018
(73) Titulaire: Pharma Technology S.A., 1400 Nivelles (BE)
(72) Inventeur: DOLLINGER, Martial, 1420 Braine l'Alleud (BE)
(74) Mandataire: Gevers Patents
(86) Numéro de dépôt international: PCT/EP2015/000601
(87) Numéro de publication internationale: WO 2016/146141

(56) Documents cités:
- WO-A1-95/19838
- FR-A1- 2 258 895
- FR-A2- 2 311 583
- US-A- 366 820
- US-A1- 2012 189 748

## Description

La présente invention concerne un dispositif d'enrobage de matériau particulaire, notamment de comprimés pharmaceutiques.

De tels dispositifs sont utilisés pour appliquer sur un matériau particulaire, une couche superficielle d'un agent d'enrobage destiné à protéger les particules enrobées d'éventuels polluants extérieurs ou à préserver leur intégrité physique. L'agent d'enrobage peut contenir un colorant et/ou une substance conférant aux particules enrobées une dureté et/ou un lissage améliorés ainsi qu'une substance différant dans le temps la libération du principe actif d'un comprimé de médicament. Lorsque de tels dispositifs d'enrobage sont utilisés dans l'industrie pharmaceutique pour enrober des comprimés de médicaments, un dispositif d'enrobage est généralement disposé dans une zone située entre une zone où se trouve un dispositif de dépoussiérage des comprimés après leur fabrication à partir de poudre de médicament compressée et une zone où se trouve un dispositif de conditionnement des comprimés enrobés dans un emballage approprie.

Différents types de dispositifs d'enrobage de matériau particulaire et notamment de comprimés pharmaceutiques existent selon l'état de la technique : Ainsi, la demande de brevet US 2010/0092694 A1 concerne un dispositif d'enrobage de comprimés pharmaceutiques comprenant une enceinte verticale au sein de laquelle est agencée une tour cylindrique comprenant une colonne centrale à laquelle est fixée une rampe hélicoïdale continue de transport des comprimés à enrober depuis la base de la tour où ils sont introduits dans le dispositif jusqu'à son sommet où ils en sont évacués après enrobage. Le mouvement ascendant des comprimés dans la tour est obtenu par vibration de celle-ci. Au cours de leur ascension le long de la rampe de la tour, les comprimés sont enrobés d'agent d'enrobage pulvérisé par des moyens de pulvérisation (sprays) orientes vers ladite rampe.

Un tel dispositif présente l'inconvénient que dès lors que le matériau particulaire à enrober est déplacé dans le dispositif de manière ascendante pendant que de l'agent d'enrobage est pulvérisé dessus, le mouillage du matériau par l'agent d'enrobage le ralentit dans son ascension car les particules mouillées « patinent » sur la rampe de la tour, ce qui occasionne des encombrements localisés de ce matériau particulaire, de tels encombrements étant défavorables à un enrobage optimal dès lors que dans ces zones d'encombrement, le matériau particulaire reçoit trop d'agent d'enrobage et les particules enrobées s'y collent les unes aux autres. Par ailleurs, dans un dispositif selon la demande de brevet en question, les particules à enrober ont tendance à ne pas être exposées à l'agent d'enrobage de manière uniforme sur toute leur surface car elles tendent à conserver leur orientation initiale lors de leur ascension.

La demande de brevet US 366 820 résout ce problème en proposant un dispositif de pulvérisation d'eau sur des particules rocheuses afin de laver ces particules, dans lequel elles se déplacent par gravité sur des tables planes inclinées disposées les unes en dessous des autres et sont exposées à la pulvérisation lors de leur chute d'une table à celle lui étant sous-jacente.

Un tel dispositif présente l'inconvénient d'être très encombrant. En effet, les particules s'y déplacent par gravité et dès lors, il est nécessaire que les tables inclinées qu'il comprend le soient suffisamment que pour assurer le déplacement en question, ce qui entraine une hauteur importante du dispositif pour un nombre de zones de pulvérisation donné. Par ailleurs, le dispositif en question présente une structure complexe. En effet, un châssis périphérique au dispositif doit être prévu pour que chacune des tables susmentionnées y soit fixée.

WO 95/19838 répond au problème d'encombrement susmentionné en proposant un dispositif d'enrobage de particules d'agent fertilisant dans lequel des particules se déplacent par gravité sur des tables planes faiblement inclinées disposées les unes en dessous des autres et sont enrobées d'agent fertilisant lors de leur chute d'une table à celle lui étant sous-jacente, par des moyens de pulvérisation (sprays) disposés au droit de la zone de chute desdites particules. Pour assurer un déplacement correct des particules alors que les tables qu'il comprend sont faiblement inclinées, le dispositif décrit demande qu'un moyen de fluidisation desdites particules soit prévu, constitué d'injecteurs d'air disposés sous chaque table pour projeter de l'air au travers d'orifices agences dans ces tables de sorte à y fluidiser les particules en question pour qu'elles aboutissent correctement dans la zone de chute dans laquelle elles sont enrobées d'agent fertilisant. Le dispositif en question présente donc l'inconvénient de requérir une structure encore plus complexe que celle du dispositif selon US 366 820.

La présente invention palie les inconvénients susmentionnés en proposant un dispositif d'enrobage de matériau particulaire, comprenant une colonne centrale agencée pour être disposée verticalement, un moyen d'alimentation du dispositif en matériau particulaire à enrober et un moyen d'évacuation de matériau particulaire enrobé, le dispositif comprenant une rampe de convoyage connectée à la colonne centrale et une pluralité de moyens de pulvérisation d'un agent d'enrobage, chaque moyen de pulvérisation étant agencé pour orienter ladite pulvérisation vers ledit matériau particulaire lors de son convoyage dans le dispositif. Le moyen d'alimentation est agencé au sommet du dispositif et le moyen d'évacuation est agencé à la base du dispositif et la rampe de convoyage comprend une pluralité de sections de rampe agencées les unes au-dessus des autres de sorte à définir entre deux sections successives une zone de chute pour ledit matériau particulaire entre l'une et l'autre desdites sections successives.

Grâce au fait que le moyen d'alimentation du dispositif est agencé au sommet de ce dernier, que son moyen d'évacuation est agencé à sa base et au fait que la rampe de convoyage comprend une pluralité de sections de rampe agencées les unes au-dessus des autres de sorte à définir entre deux sections successives une zone de chute pour le matériau particulaire entre l'une et l'autre desdites sections successives, les particules sont transportées dans le dispositif sans que des zones d'accumulations de ces particules n'y apparaissent dès lors que mêmes mouillées par de l'agent d'enrobage, les particules de matériau transporté dans le dispositif ne « patinent » pas sur sa rampe car elles sont aidées dans leur descente de celle-ci par la gravité. Par ailleurs, l'orientation de ces particules est modifiée au travers des zones de chute de cette rampe ce qui permet qu'elles soient exposées sur toute leur surface à la pulvérisation et dès lors d'obtenir un enrobage uniforme desdites particules.

L'invention va à présent être décrite plus en détails à partir d'un exemple de mode de réalisation non limitatif, illustré par les figures jointes, dans lesquelles:

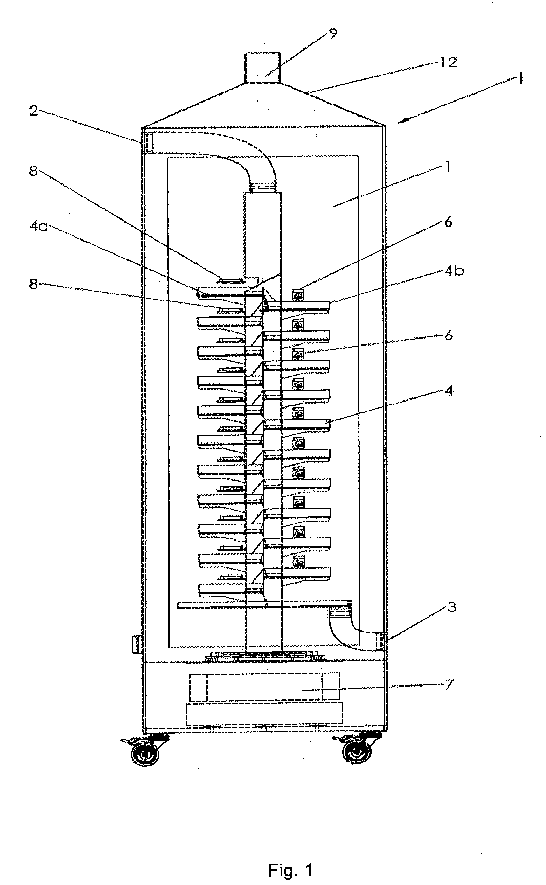

La figure 1 est une vue de face d'un mode de réalisation de l'invention;

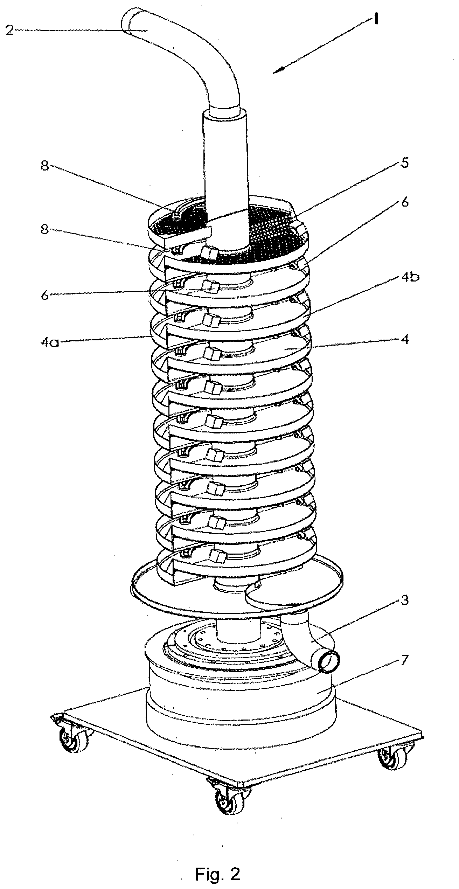

La figure 2 est une vue en perspective du mode de réalisation de l'invention illustré à la figure 1, sans son enceinte de confinement.

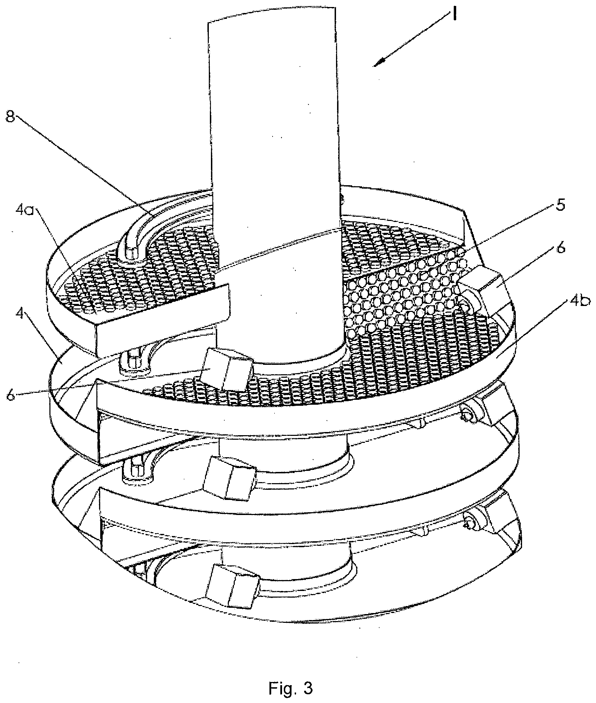

La figure 3 est un agrandissement de la partie supérieure de la vue de la figure 2.

En se rapportant aux figures, il y est illustré un dispositif I d'enrobage de matériau particulaire, comprenant une colonne centrale 1 agencée pour être disposée verticalement, un moyen d'alimentation 2 du dispositif en matériau particulaire à enrober et un moyen d'évacuation 3 de matériau particulaire enrobé, le dispositif comprenant une rampe de convoyage 4 connectée à la colonne centrale et une pluralité de moyens de pulvérisation 6 d'un agent d'enrobage, chaque moyen de pulvérisation étant agencé pour orienter ladite pulvérisation vers ledit matériau particulaire lors de leur convoyage dans le dispositif.

Le moyen d'alimentation est agencé au sommet du dispositif et le moyen d'évacuation à sa base de sorte que le matériau particulaire est convoyé dans ce dispositif sans que ne s'y créent de zones d'accumulation de ce matériau, grâce à l'effet de la gravité sur ce dernier.

La rampe de convoyage comprend une pluralité de sections de rampe 4a, 4b agencées les unes au-dessus des autres de sorte à définir entre deux sections successives une zone de chute 5 pour ledit matériau particulaire entre l'une et l'autre desdites sections successives. Deux sections successives peuvent être liées entre elles comme dans le mode de réalisation illustré par des éléments de connexion. Alternativement, dans un mode de réalisation non illustré de l'invention, une section supérieure à la section inférieure successive peut légèrement chevaucher cette dernière de sorte que les particules de matériau particulaire transporté dans le dispositif tombent toutes correctement de la section supérieure sur la section inférieure.

Le dispositif comprend encore un moyen 7 agencé pour animer la rampe d'un mouvement oscillant ou vibratoire pour convoyer le matériau particulaire entre le moyen d'alimentation et le moyen d'évacuation du dispositif. Dans le mode de réalisation illustré, ce moyen est constitué d'un moteur oscillant agencé sous la colonne centrale et connecté à cette dernière de sorte à pouvoir l'animer dans son entièreté du mouvement oscillant ou vibratoire désiré et de la sorte animer également du même mouvement la rampe du dispositif connectée à cette colonne centrale. De cette manière, les comprimés sont entrainés du moyen d'alimentation au moyen d'évacuation du dispositif grâce à ce mouvement en plus de l'effet de la gravité et ce par un seul et même moyen, ce qui confère audit dispositif une structure simple et pratique.

Dans le mode de réalisation illustré, chaque section de rampe est plane et agencée orthogonalement à la colonne centrale de sorte à être disposées horizontalement lorsque la colonne est verticale. Cette configuration permet d'obtenir un dispositif très compact en raison de la faible hauteur occupée par deux sections de rampe successives. Alternativement, des sections de rampe obliques relativement à la colonne centrale peuvent être utilisée, ce qui permet d'augmenter la vitesse de convoyage de matériau particulaire dans le dispositif en l'y soumettant davantage à l'effet d'entrainement par gravité entre le sommet et la base dudit dispositif.

Dans le mode de réalisation illustré les sections de rampe présentent un bord extérieur en arc de cercle, ce qui permet que le matériau particulaire qui y est convoyé l'y soit avec la même vitesse angulaire sur tout le rayon de telles sections de rampe, ce qui favorise une répartition optimale dudit matériau sur chaque section de rampe et évite que des zones où du matériau particulaire s'accumule ne s'y forment en raison de vitesses angulaires différentes de ces particules. Lorsque les sections de rampe ne sont pas orthogonales mais obliques relativement à la colonne centrale du dispositif elles peuvent néanmoins s'inscrire dans un cylindre et conserver de la sorte l'avantage susmentionné en termes de vitesse angulaire constante, en particulier lorsque ces sections de rampe sont à forme de section d'hélicoïde.

Le dispositif comprend également une pluralité de moyens de séchage 8 du matériau particulaire enrobé, constitués dans la forme de réalisation illustrée de lampes radiantes à infrarouges, chaque telle lampe étant orientée en direction d'une section de rampe différente pour que l'enrobage du matériau particulaire qui y est convoyé soit séché durant le transit de ce matériau particulaire enrobé sur ladite section de rampe. Tout autre moyen de séchage connu de l'homme du métier pourrait alternativement être utilisé dans le cadre de l'invention. En pratique, comme illustré dans les figures, les moyens de pulvérisation et de séchage sont disposés en alternance au-dessus de sections de rampe successives, un moyen de pulvérisation surplombant une première section de rampe et un moyen de séchage surplombant la section de rampe qui est sous-jacente à ladite première section.

Le dispositif comprend encore une enceinte de confinement 10 agencée pour isoler les éléments constitutifs du dispositif de l'extérieur de celui-ci de sorte que de l'agent d'enrobage pulvérisé ne s'en échappe pas et que l'enrobage ne soit pas perturbé par des courants d'air ambiant, ainsi qu'un moyen 9 de renouvellement de l'air contenu dans le dispositif, de sorte que cet air ne devienne pas excessivement humide en raison de la projection au sein du dispositif d'agent d'enrobage. Ce moyen peut par exemple être constitué d'un ventilateur disposé sous ou au-dessus de la rampe du dispositif et insufflant de l'air au travers de celui-ci.

En pratique, le dispositif illustré est utilisé pour l'enrobage de matériau particulaire constitué de comprimés pharmaceutiques mais peut également servir à enrober d'autres matériaux particulaires.

## Revendications

1. Dispositif (I) d'enrobage de matériau particulaire constitué de comprimés pharmaceutiques, comprenant une colonne centrale (1) agencée pour être disposée verticalement, un moyen d'alimentation (2) du dispositif en matériau particulaire à enrober et un moyen d'évacuation (3) de matériau particulaire enrobé, celui-ci comprenant une rampe de convoyage (4) connectée à la colonne centrale et une pluralité de moyens de pulvérisation (6) d'un agent d'enrobage, chaque moyen de pulvérisation étant agencé pour orienter ladite pulvérisation vers ledit matériau particulaire lors de son convoyage dans le dispositif, **caractérisé en ce que** ledit moyen d'alimentation est agencé au sommet du dispositif et ledit moyen d'évacuation est agencé à la base dudit dispositif et **en ce que** ladite rampe de convoyage comprend une pluralité de sections de rampe agencées les unes au-dessus des autres de sorte à définir entre deux sections successives (4a, 4b) une zone de chute (5) pour ledit matériau particulaire entre l'une et l'autre desdites sections successives.

2. Dispositif selon la revendication 1, **caractérisé en ce qu'**il comprend un moyen (7) agencé pour animer la rampe d'un mouvement oscillant ou vibratoire pour convoyer le matériau particulaire entre le moyen d'alimentation et le moyen d'évacuation du dispositif.

3. Dispositif selon la revendication 2, **caractérisé en ce que** chaque section de rampe est plane et agencée orthogonalement à la colonne centrale.

4. Dispositif selon la revendication 3, **caractérisé en ce que** chaque section de rampe présente un bord extérieur en arc de cercle.

5. Dispositif selon l'une quelconque des revendications précédentes **caractérisé en ce qu'**il comprend une pluralité de moyens de séchage (8) du matériau particulaire enrobé.

6. Dispositif selon l'une quelconque des revendications précédentes, **caractérisé en ce qu'**il comprend un moyen de renouvellement (9) de l'air contenu dans le dispositif.

## Patentansprüche

1. Vorrichtung (I) zum Beschichten von Partikelmaterial, das aus pharmazeutischen Tabletten besteht, umfassend eine zentrale Säule (1), die angeordnet ist, um vertikal bereitgestellt zu werden, ein Mittel zum Versorgen (2) der Vorrichtung mit Partikelmaterial, das beschichtet werden soll, und ein Mittel zum Abführen (3) von beschichtetem Partikelmaterial, wobei dieses eine Förderrampe (4), die mit der zentralen Säule verbunden ist, und eine Vielzahl von Sprühmitteln (6) einer Beschichtungssubstanz umfasst, wobei jedes Sprühmittel angeordnet ist, um das Sprühen zum Partikelmaterial bei seiner Förderung in der Vorrichtung auszurichten, **dadurch gekennzeichnet, dass** das Mittel zum Versorgen am Kopfende der Vorrichtung angeordnet ist und das Mittel zum Abführen am Fußende der Vorrichtung angeordnet ist, und dadurch, dass die Förderrampe eine Vielzahl von Rampenabschnitten umfasst, die so übereinander angeordnet sind, dass sie zwischen zwei aufeinanderfolgenden Abschnitten (4a, 4b) einen Fallbereich (5) für das Partikelmaterial zwischen dem einen und dem anderen der aufeinanderfolgenden Abschnitte definieren.

2. Vorrichtung nach Anspruch 1, **dadurch gekennzeichnet, dass** sie ein Mittel (7) umfasst, das angeordnet ist, um der Rampe eine schwingende oder vibrierende Bewegung zu verleihen, um das Partikelmaterial zwischen dem Mittel zum Versorgen und dem Mittel zum Abführen der Vorrichtung zu befördern.

3. Vorrichtung nach Anspruch 2, **dadurch gekennzeichnet, dass** jeder Rampenabschnitt eben ist und orthogonal zur zentralen Säule angeordnet ist.

4. Vorrichtung nach Anspruch 3, **dadurch gekennzeichnet, dass** jeder Rampenabschnitt einen kreisbogenförmigen äußeren Rand aufweist.

5. Vorrichtung nach einem der vorstehenden Ansprüche, **dadurch gekennzeichnet, dass** sie eine Vielzahl von Mittel zum Trocknen (8) des beschichteten Partikelmaterials umfasst.

6. Vorrichtung nach einem der vorstehenden Ansprüche, **dadurch gekennzeichnet, dass** sie ein Mittel zum Erneuern (9) der in der Vorrichtung enthaltenen Luft umfasst.

## Claims

1. Device (I) for coating particulate material consisting of pharmaceutical tablets, comprising a central column (1) arranged so as to be disposed vertically, a supply means (2) for supplying the device with particulate material to be coated and a discharge means (3) for discharging coated particulate material, the device comprising a conveying ramp (4) connected to the central column and a plurality of spray means (6) for spraying a coating agent, each spray means being arranged so as to orient said spray towards said particulate material while it is conveyed in the device, **characterised in that** said supply means is arranged at the top of the device and said discharge means is arranged at the base of said device and **in that** said conveying ramp comprises a plurality of ramp sections arranged one above the other so as to define, between two successive sections (4a, 4b), a drop zone (5) for said particulate material to fall between each of said successive sections.

2. Device according to claim 1, **characterised in that** it comprises a means (7) arranged to impart an oscillating or vibratory movement to the ramp in order to convey the particulate material between the supply means and the discharge means of the device.

3. Device according to claim 2, **characterised in that** each ramp section is planar and arranged orthogonally to the central column.

4. Device according to claim 3, **characterised in that** each ramp section has an outer edge in an arc of a circle.

5. Device according to any of the preceding claims, **characterised in that** it comprises a plurality of drying means (8) for drying coated particulate material.

6. Device according to any of the preceding claims, **characterised in that** it comprises a means (9) for renewing the air contained in the device.
